# EUROPEAN PATENT APPLICATION

(11) **EP 2 157 077 A1**
(43) Date of publication of application: **24.02.2010**
(21) Application number: 08777111.9
(22) Date of filing: 06.06.2008
(51) Int. Cl.: C07C 209/10, C07C 211/54, C07C 211/58, C07B 61/00

(54) **METHOD FOR PRODUCING TRIARYLAMINE COMPOUND**

(30) Priority: 06.06.2007 JP 2007150100
(71) Applicant: Hodogaya Chemical Co., Ltd., Tokyo 105-0011 (JP)
(72) Inventor: KIMURA, Ikuo, Tsukuba-shi Ibaraki 305-0841 (JP)
(74) Representative: Müller-Boré & Partner Patentanwälte
(86) International application number: PCT/JP2008/060491
(87) International publication number: WO 2008/149993

(57) **Abstract**

[Problem] To provide a method for producing a triarylamine compound in which the reaction can be attained under a practical condition, the purification operation after the reaction is simple, the environmental burden is reduced and the production efficiency is high.

[Means for Resolution] A method for producing a triarylamine compound through amination of a diarylamine compound and an aryl halide, wherein a catalyst comprising a salt of an imidazolium derivative represented by the following general formula (1) and a palladium compound is used, as combined with a base and a solvent to coexist therein.

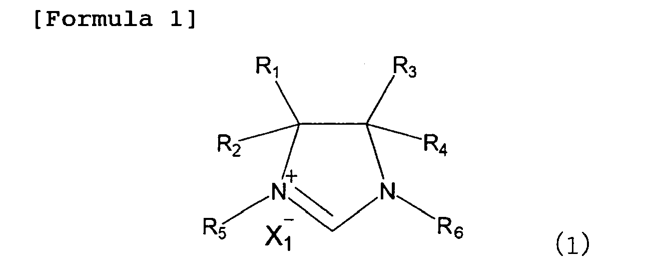

## Description

### TECHNICAL FIELD

The present invention relates to a method for producing a triarylamine compound. The triarylamine compound is a compound extremely important in the field of organic functional materials such as organic EL materials, organic electroconductive materials, etc.

### BACKGROUND ART

For a method of producing a triarylamine compound from a diarylamine compound and an aryl halide, a method of using copper catalyst is known as Ullmann-Goldberg reaction (for example, see Non-Patent Reference 1). However, the method requires using a large amount of a copper catalyst, and removing it is a great burden to purification operation and is a bar to practicability. In addition, the method is characterized by requiring a high reaction temperature, and the product is significantly colored and a large quantity of side products are produced, and therefore the method is defective in that the yield of arylamines is generally low.

Also reported is a method of using a palladium compound as a catalyst further with a trialkyl phosphine as a ligand (for example, see Patent Reference 1). The condition has the advantage of using an inexpensive aryl chloride as the starting material, but requires a strict inert gas atmosphere for the reaction for maintaining the activity of the palladium-phosphine complex. In addition, since the trialkyl phosphine itself to be a ligand is unstable in air, it must be stored and metered in an inert gas atmosphere and its practicability is poor.
Further reported is a method of using an heterogeneous palladium catalyst and, as a ligand, a phosphine-based ligand such as 1,1'-bis(diphenylphosphino)ferrocene (for example, see Patent Reference 2). Under the condition, an inexpensive aryl chloride can be used as the starting material, and the method is advantageous in that a strict inert gas atmosphere is unnecessary for maintaining the activity of the palladium-phosphine complex; however, since the activity of the heterogeneous palladium catalyst is low, the method requires an aryl bromide or an aryl iodide of high reactivity and requires a high reaction temperature of not lower than 180°C, and its practicability is poor.

Reported is a reaction of using, as a ligand, a carbene compound formed by the use of an imidazolium salt in the presence of a base, in place of using a phosphine compound as a ligand (for example, see Patent References 3 and 4 and Non-Patent Reference 2). The reaction condition is **characterized in that** the ligand is provided as an imidazolium salt that is a precursor thereof. The compound is stable and can be stored and handled in air, and is therefore extremely effective under practical production conditions. However, for producing an arylamine from an inexpensive aryl chloride and an amine as starting materials, a 0-valent palladium complex such as bis(dibenzylideneacetone)palladium or the like must be used as the palladium compound. Some other types of 0-valent palladium complexes are known in addition to the above, all of which, however, are extremely expensive and are unsuitable to practical use.

Patent Reference 1: JP-A 10-139742
Patent Reference 2: JP-A 2006-335712
Patent Reference 3: JP-A 2002-284745
Patent Reference 4: JP-A 2004-262891
Non-Patent Reference 1: Tetrahedron, 40, 1433 (1984)
Non-Patent Reference 2: J. Org. Chem., Vol. 66, 7729 (2001)

### DISCLOSURE OF THE INVENTION

### PROBLEMS THAT THE INVENTION IS TO SOLVE

The present invention is to provide a method for producing a triarylamine compound in which the reaction can be attained under a practical condition, the purification operation after the reaction is simple, the environmental burden is reduced and the production efficiency is high.

### MEANS FOR SOLVING THE PROBLEMS

The present inventors have assiduously studied for the purpose of solving these problems and, as a result, have completed a production method for an amination of a diarylamine compound and an aryl halide in the presence of a base, using a catalyst that comprises a salt of an imidazolium derivative having a specific structure and an inexpensive palladium compound, in which the production efficiency is high and which is applicable to industrial-scale production and reduces an environmental burden.

Specifically, the invention is a method for producing a triarylamine compound through amination of a diarylamine compound and an aryl halide, wherein a catalyst comprising a salt of an imidazolium derivative represented by the following general formula (1):

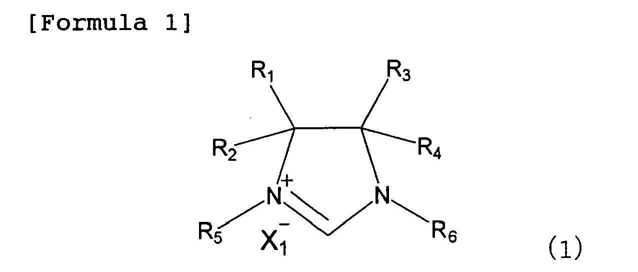

(wherein R1, R2, R3, R4, R5 and R6 may be the same or different, each representing a hydrogen atom or a hydrocarbon group having from 1 to 20 carbon atoms; X1 represents a chlorine atom, a bromine atom, an iodine atom, BF₄, CF₃SO₃ or PF₆) and a palladium compound is used, as combined with a base and a solvent to coexist therein; and preferably, the base and the solvent coexist with no other additive as combined therein.

Also, the invention is a method for producing a triarylamine compound through amination of a diarylamine compound and an aryl halide, wherein a catalyst comprising a salt of an imidazolium derivative represented by the following general formula (2):

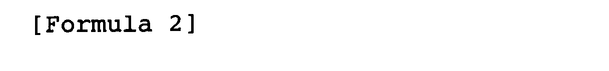

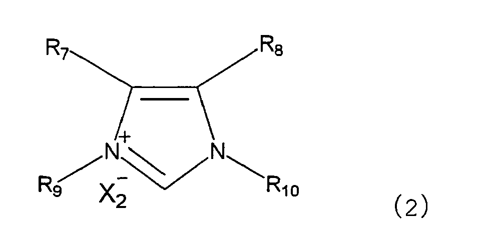

(wherein R7 and R8 may be the same or different, each representing a hydrogen atom, a hydrocarbon group having from 1 to 20 carbon atoms, or an alkoxy group having from 1 to 20 carbon atoms; R9 and R10 may be the same or different, each representing a hydrogen atom, or a hydrocarbon group having from 1 to 20 carbon atoms; R7 and R9 may form a ring, as taken together, and R8 and R10 may form a ring, as taken together; X2 represents a chlorine atom, a bromine atom, an iodine atom, BF₄, CF₃SO₃ or PF₆) and a palladium compound is used, as combined with a base and a solvent to coexist therein; and preferably, the base and the solvent coexist with no other additive as combined therein.

The hydrocarbon group having from 1 to 20 carbon atoms for R1 to R6 in the above-mentioned general formula (1) includes a saturated aliphatic hydrocarbon group, an unsaturated aliphatic hydrocarbon group, an alicyclic hydrocarbon group, an alicyclic-aliphatic hydrocarbon group, an aromatic hydrocarbon group, an aromatic-aliphatic hydrocarbon group, etc.

Examples of the saturated aliphatic hydrocarbon group include, for example, methyl, ethyl, n-propyl, isopropyl, n-butyl, isobutyl, tert-butyl, n-pentyl, isopentyl, neopentyl, tert-pentyl, 2-methylbutyl, n-hexyl, isohexyl, 3-methylpentyl, ethylbutyl, n-heptyl, 2-methylhexyl, n-octyl, isooctyl, tert-octyl, 2-ethylhexyl, 3-methylheptyl, n-nonyl, isononyl, 1-methyloctyl, ethylheptyl, n-decyl, 1-methylnonyl, n-undecyl, 1,1-dimethylnonyl, n-dodecyl, n-tetradecyl, n-heptadecyl, n-octadecyl groups, etc.

Suitable specific examples of the unsaturated aliphatic hydrocarbon group include, for example, vinyl, allyl, isopropenyl, 2-butenyl, 2-methylallyl, 1,1-dimethylallyl, 3-methyl-2-butenyl, 3-methyl-3-butenyl, 4-pentenyl, hexenyl, octenyl, nonenyl, decenyl groups, etc.

Suitable specific examples of the alicyclic hydrocarbon group include, for example, cyclopropyl, cyclobutyl, cyclopentyl, cyclohexyl, cycloheptyl, cyclooctyl, 3-methylcyclohexyl, 4-methylcyclohexyl, 4-ethylcyclohexyl, 2-methylcyclooctyl, cyclopropenyl, cyclobutenyl, cyclopentenyl, cyclohexenyl, cyclooctenyl, 4-methylcyclohexenyl, 4-ethylcyclohexenyl, 1-adamantyl groups, etc.

Suitable specific examples of the alicyclic-aliphatic hydrocarbon group include, for example, alkyl and alkenyl groups and the like substituted with cyclopropylethyl, cyclobutylethyl, cyclopentylethyl, cyclopentylmethyl, cyclohexylmethyl, cyclohexylethyl, cycloheptylmethyl, cyclooctylmethyl, 3-methylcyclohexylpropyl, 4-methylcyclohexylethyl, 4-ethylcyclohexylethyl, 2-methylcyclooctylethyl, cyclopropenylbutyl, cycloheptenylethyl, cyclopentenylethyl, cyclohexenylmethyl, cycloheptenylmethyl, cyclooctenylethyl, 4-methylcyclohexenylpropyl, 4-ethylcyclohexenylpentyl groups, etc., alkynyl group.

Suitable specific examples of the aromatic hydrocarbon group include, for example, aryl groups such as phenyl, naphthyl groups, etc.; alkylaryl groups such as 4-methylphenyl, 3,4-dimethylphenyl, 2,6-diisopropylphenyl, 2,4,6-trimethylphenyl, 3,4,5-trimethylphenyl, 2-ethylphenyl, n-butylphenyl, tert-butylphenyl, amylphenyl, hexylphenyl, nonylphenyl, 2-tert-butyl-5-methylphenyl, cyclohexylphenyl, cresyl, oxyethylcresyl, 2-methoxy-4-tert-butylphenyl, dodecylphenyl groups, etc., alkenylaryl group and alkynylaryl group. The alkyl moiety of the alkylaryl group, the alkenyl moiety of the alkenylaryl group, and the alkynyl moiety of the alkynylaryl group may have a cyclic structure.

Specific examples of the aromatic-aliphatic hydrocarbon group include, for example, aralkyl groups such as benzyl, 1-phenylethyl, 2-phenylethyl, 2-phenylpropyl, 3-phenylpropyl, 4-phenylbutyl, 5-phenylpentyl, 6-phenylhexyl, 1-(4-methylphenyl)ethyl, 2-(4-methylphenyl)ethyl, 2-methylbenzyl, 1,1-dimethyl-2-phenylethyl groups, etc., aralkenyl group and aralkynyl group. The alkyl moiety of the aralkyl group, the alkenyl moiety of the aralkenyl group, and the alkynyl moiety of the aralkynyl group may have a cyclic structure.

The hydrocarbon group having from 1 to 20 carbon atoms represented by R7 to R10 in the above-mentioned formula (2) include the same as those of the hydrocarbon group having from 1 to 20 carbon atoms represented by R1 to R6 in the above-mentioned formula (1).

The alkoxy group having from 1 to 20 carbon atoms represented by R7 and R8 in the above-mentioned general formula (2) includes methoxy, ethoxy, n-propoxy, 2-propoxy, n-butoxy, isobutoxy, tert-butoxy, n-pentyloxy, isopentyloxy, neopentyloxy, tert-pentyloxy, 2-methylbutoxy, n-hexyloxy, isohexyloxy, 3-methylpentyloxy, n-heptyloxy, 2-methylhexyloxy, n-octyloxy, isooctyloxy, tert-octyloxy, 2-ethylhexyloxy, 3-methylheptyloxy, n-nonyloxy, isononyloxy, 1-methyloctyloxy, ethylheptyloxy, n-decyloxy, 1-methylnonyloxy, n-undecyloxy, 1,1-dimethylnonyloxy, n-dodecyloxy, n-tetradecyloxy, n-heptadecyloxy, n-octadecyloxy groups, etc.

### ADVANTAGE OF THE INVENTION

In the invention, an industrially-applicable triarylamine compound can be produced with good production efficiency, using an inexpensive aryl halide as the starting material and using an inexpensive palladium compound as the catalyst; and the reaction may be attained under a practical condition with no additive to coexist during the reaction, and the purification operation after the reaction is simple. Since trialkyl phosphines are not used, it is possible to produce triarylamine compounds with reducing the burden to the environment by trialkyl phosphines that are phosphorus compounds.

### BEST MODE FOR CARRYING OUT THE INVENTION

Specific examples of the compounds of general formula (1) or (2) for use in the invention include, for example, those having the following structural formulae, to which, however, the invention is not limited.

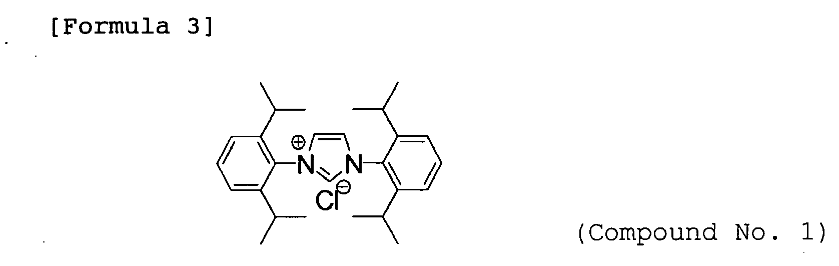

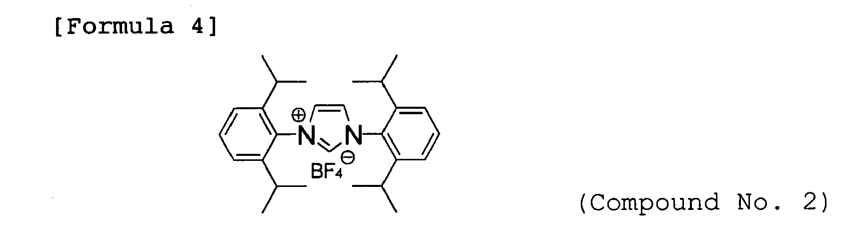

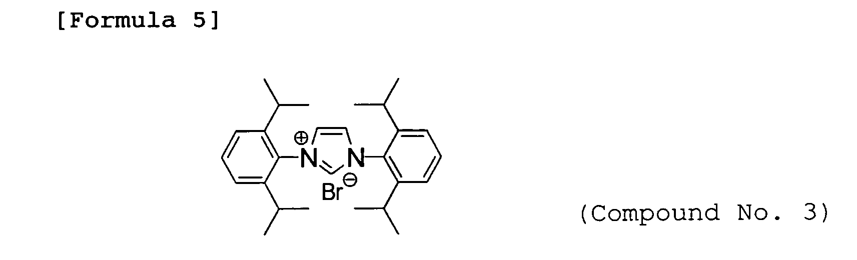

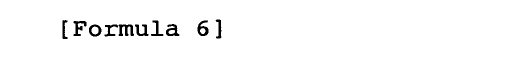

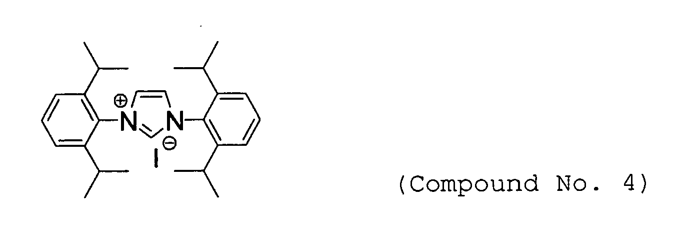

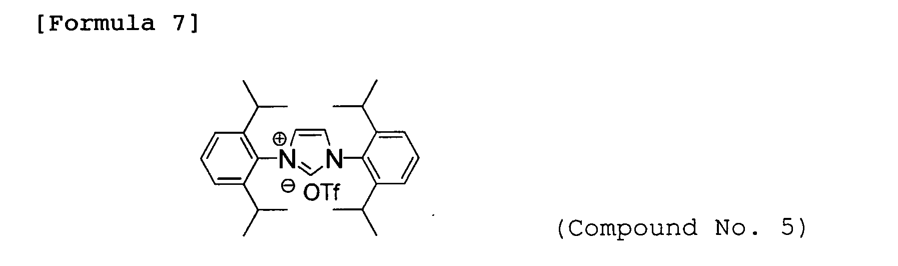

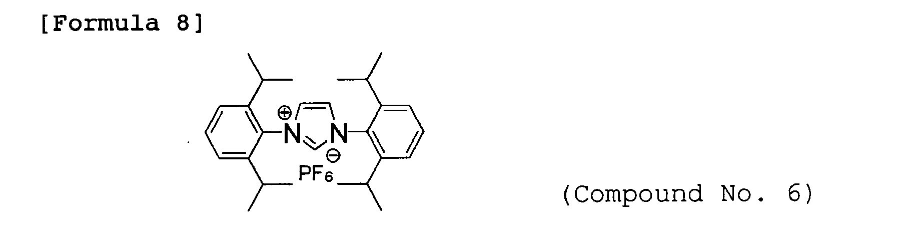

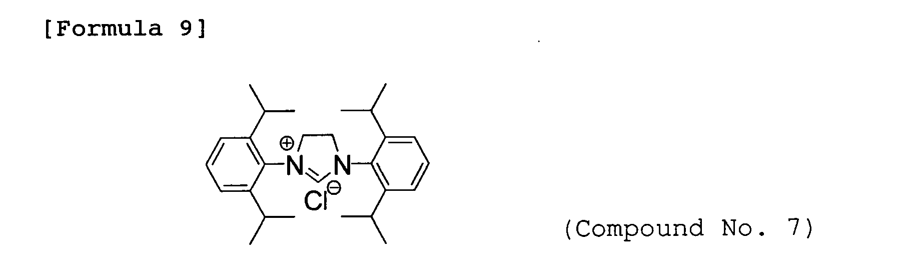

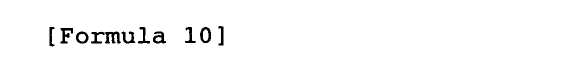

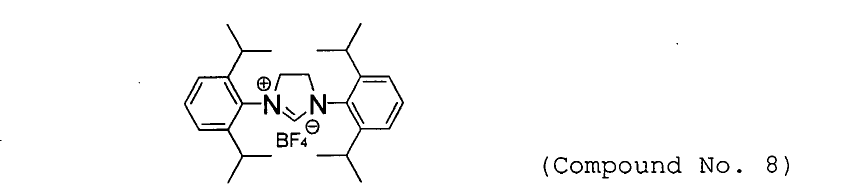

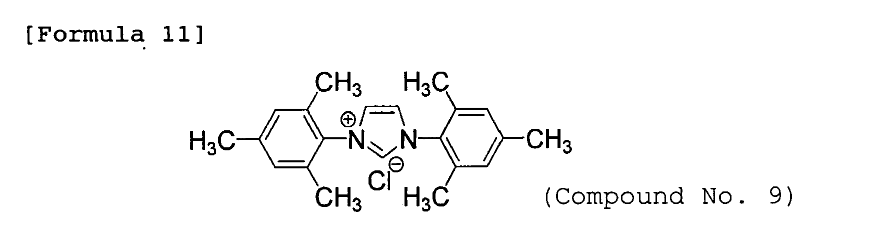

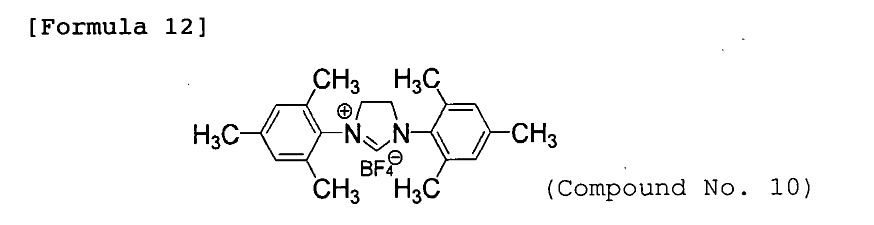

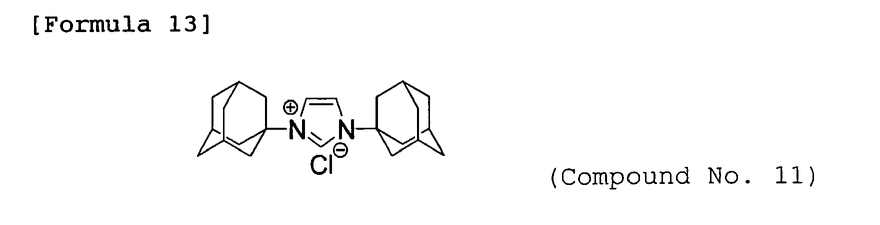

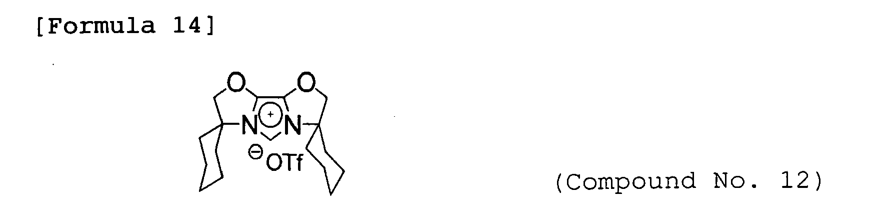

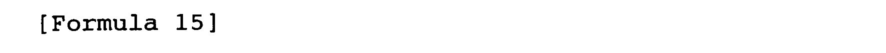

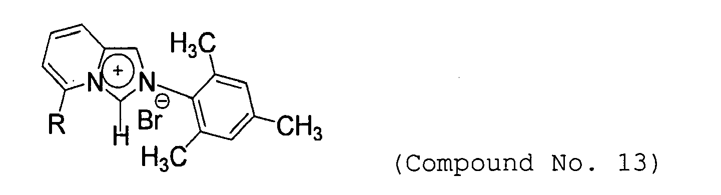

In the invention, the base for use in the reaction is not specifically defined and may be any one selected from inorganic bases or organic bases. Preferred are alkali metal alkoxides such as sodium methoxide, sodium ethoxide, potassium methoxide, potassium ethoxide, lithium tert-butoxide, sodium tert-butoxide, potassium tert-butoxide, etc. The base may be directly added to the reaction solution, but may be prepared in situ from an alkali metal, alkali metal hydride, alkali metal hydroxide or the like and added to the reaction solution.

In the invention, the amount of the base to be used in the reaction is preferably at least 0.5 molar equivalent the hydrogen halide to be produced in the reaction, more preferably within a range of from 1 to 3 molar equivalent. When the amount of the base to be used in the reaction is less than 0.5 molar equivalent, then it is unfavorable since the yield of the triarylamine to be produced may be low. However, even when a large excessive amount of the base is added to the reaction, it has no influence on the yield of the triarylamine to be produced but it is unfavorable since the post treatment operation after the end of the reaction is complicated.

Not specifically defined, the diarylamine compound to be used in the invention includes diphenylamine, naphthylphenylamine, dinaphthylamine, anthrylphenylamine, phenanthrylphenylamine, pyrenylphenylamine, bis(4'-diphenylaminophenyl-4-yl)amine, N,N'-diphenylbenzidine, 4,4"-bis(phenylamino)-p-terphenyl, 1,3,5-tris(phenylamino)benzene, N,N-bis(4'-diphenylaminophenyl-4-yl)amine, N,N-bis(4'-diphenylaminobiphenyl-4-yl)amine, 1,3,5-tris(4'-phenylaminophenyl-4-yl)benzene, N,N,N-tris(4'-phenylaminophenyl-4-yl)amine, N,N,N-tris(4"-phenylaminobiphenyl-4-yl)amine; and the aryl group in the diarylamine may have a substituent. The substituent for the aryl group in the diarylamine includes a halogen atom, a linear or branched alkyl group having from 1 to 4 carbon atoms, such as methyl, ethyl, tert-butyl groups; a linear or branched alkoxy group having from 1 to 4 carbon atoms such as methoxy, ethoxy, tert-butoxy groups; a diarylamino group such as a diphenylamino group, a dinaphthylamino group, a dianthrylamino group, a diphenanthrylamino group, a dipyrenylamino group, etc.; an aryl group such as phenyl, biphenyl, naphthyl, anthryl, phenanthryl, pyrenyl groups, etc. The diarylamino group and the aryl group may further have a substituent. The substituent for the aryl group in the diarylamino group includes a halogen atom, a linear or branched alkyl group having from 1 to 4 carbon atoms, such as methyl, ethyl, tert-butyl groups; a linear or branched alkoxy group having from 1 to 4 carbon atoms such as methoxy, ethoxy, tert-butoxy groups; a diarylamino group such as a diphenylamino group, a dinaphthylamino group, a dianthrylamino group, a diphenanthrylamino group, a dipyrenylamino group, etc.; an aryl group such as phenyl, biphenyl, naphthyl, anthryl, phenanthryl, pyrenyl groups, etc. The substituent for the aryl group includes a halogen atom, a linear or branched alkyl group having from 1 to 4 carbon atoms, such as methyl, ethyl, tert-butyl groups; a linear or branched alkoxy group having from 1 to 4 carbon atoms such as methoxy, ethoxy, tert-butoxy groups; a diarylamino group such as a diphenylamino group, a dinaphthylamino group, a dianthrylamino group, a diphenanthrylamino group, a dipyrenylamino group, etc.; and the diarylamino group may further have a substituent.

Not specifically defined, the aryl halide for use in the invention includes benzene halides, naphthalene halides, anthracene halides, phenanthrene halides, pyrene halides, etc. The halogen atom in the aryl halide for use in the invention may be any of fluorine, chloride, bromine and iodide, but is preferably chlorine or bromine, more preferably bromine.

The aryl halide for use in the invention may have a substituent in the aromatic ring thereof, in addition to the halogen atom therein. The substituent includes a trifluoromethyl group; a linear or branched alkyl group having from 1 to 4 carbon atoms such as methyl, ethyl, tert-butyl groups; a linear or branched alkoxy group having from 1 to 4 carbon atoms such as methoxy, ethoxy, tert-butoxy groups; an aryl group such as phenyl, biphenylyl, naphthyl, anthryl, phenanthryl, pyrenyl, etc.; an aryloxy group such as phenoxy, naphthyloxy, anthryloxy, phenanthryloxy, pyrenyloxy groups, etc.; a diarylamino group such as a diphenylamino group, a dinaphthylamino group, a dianthrylamino group, a diphenanthrylamino group, a dipyrenylamino group, etc. The aryl group, the aryloxy group and the diarylamino group may further have a substituent. The substituent for the aryl group, the aryloxy group and the diarylamino group includes a halogen atom; a linear or branched alkyl group having from 1 to 4 carbon atoms such as methyl, ethyl, tert-butyl groups; a linear or branched alkoxy group having from 1 to 4 carbon atoms such as methoxy, ethoxy, tert-butoxy groups; a diarylamino group such as a diphenylamino group, a dinaphtylamino group, a dianthrylamino group, a diphenanthrylamino group, a dipyrenylamino group, etc. The diarylamino group may further have a substituent.

In the invention, the amount of the diarylamine compound to be used in the reaction is preferably within a range of from 0.05 to 20 molar equivalent relative to one mol of the halogen atom of the aryl halide participating in the reaction with it, more preferably within a range of from 0.2 to 10 molar equivalent. In case where any one compound large-excessively remains in the system after the completion of the reaction, it is unfavorable since the collection of the unreacted diarylamine compound or aryl halide may be complicated and the purification of the produced triarylamine compound may be complicated.

In the invention, the catalyst for use in the reaction may be a homogeneous catalyst or an heterogeneous catalyst; however, preferred is a homogeneous catalyst from the viewpoint that its catalyst activity is high, the reaction may go on at a lower temperature and the time necessary for the reaction may be short. Not specifically defined, the palladium compound for use in the reaction in the invention includes palladium(II) acetate, palladium(II) chloride, palladium(II) bromide, tris(dibenzylideneacetone)dipalladium(II), etc. The amount of the palladium compound to be used in the reaction in the invention is not also specifically defined as greatly varying depending on the reaction condition; but preferably, the amount falls within a range of from 0.001 to 50 mol% of the amount of the diarylamine compound to be used in the reaction, more preferably within a range of from 0.01 to 20 mol%.

The amount of the salt of an imidazole derivative of the general formula (1) or (2) to be used in the reaction in the invention is not also specifically defined as greatly varying depending on the reaction condition; but preferably, the amount falls within a range of from 0.001 to 50 mol% of the amount of the diarylamine compound to be used in the reaction, more preferably within a range of from 0.02 to 40 mol%.

Not specifically defined, the solvent for use in the invention may be any solvent inert to the reaction, and includes aromatic hydrocarbon solvents such as toluene, xylene, etc.; ether solvents such as tetrahydrofuran, 1,2-dimethoxyethane, cyclopentyl methyl ether, etc.; aliphatic hydrocarbon solvents such as heptane, octane, etc.; aprotic polar solvents such as acetonitrile, dimethylformamide, dimethylsulfoxide, etc. The solvent is preferably one that dissolves the starting materials of a diarylamine compound and an aryl halide, and the catalyst of an imidazolium derivative salt of the general formula (1) or (2) and a palladium compound.

In the invention, the reaction may be attained under any condition of normal pressure or increased pressure, but is preferably attained in an inert gas atmosphere such as nitrogen, argon or the like.

Not specifically defined in the invention, the reaction temperature may fall within a range of from 20°C to 300°C, but preferably within a range of from 50°C to 200°C. More preferred is a temperature at which the reaction may go on in a homogeneous system.

The reaction time in the invention varies depending on the type and the amount of the diarylamine compound, the aryl halide, the base, the palladium compound and the imidazolium derivative salt of the general formula (1) or (2) to be used in the reaction, and on the reaction temperature; but in general, the time preferably falls within a range of from a few minutes to 72 hours.

In the invention, the treatment after the reaction may be in any ordinary method of solvent extraction, filtration, washing or the like, thereby giving the intended triarylamine compound.

### EXAMPLES

The invention is described with reference to the following Examples, which, however, do not whatever restrict the scope of the invention.

### [Example 1]

1.5 g (15 mmol) of sodium tert-butoxide, 217 mg (0.5 mmol) of IPr-HCl (compound No. 1), 45 mg (0.2 mmol) of palladium(II) acetate, 1.7 g (10 mmol) of diphenylamine, 2.1 g (13 mmol) of bromobenzene and 20 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 7.5 hours. The reaction liquid was cooled to room temperature, and then 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give 2.6 g of a residue. The residue was purified through column chromatography (carrier, Fuji Silicia's NH silica gel 60 g; eluent, cyclohexane) to give 2.4 g of triphenylamine (yield 98%).

### [Example 2]

3.0 g (30 mmol) of sodium tert-butoxide, 217 mg (0.5 mmol) of IPr-HCl (compound No. 1), 45 mg (0.2 mmol) of palladium(II) acetate, 3.67 g (20 mmol) of 3-methyldiphenylamine, 3.2 g (10 mmol) of 4,4'-dibromobiphenyl and 30 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 7 hours. The reaction liquid was cooled to room temperature, then left overnight at room temperature, and 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 150 g; eluent, cyclohexane) to give 4.4 g of N,N'-diphenyl-N,N'-di(m-tolyl)benzidine (TPD) (yield 85.2%).

### [Example 3]

1.5 g (15 mmol) of sodium tert-butoxide, 110 mg (0.25 mmol) of IPr-HCl (compound No. 1), 26 mg (0.1 mmol) of palladium(II) acetate, 1.68 g (5 mmol) of N,N'-diphenylbenzidine, 2.56 g (12 mmol) of 1-bromonaphthalene and 30 ml of toluene were put into a 100-ml three-neck flask equipped with a stirrer, a condenser tube, a thermometer and a gas-introducing duct, in an argon current at room temperature, and reacted under reflux in an oil bath controlled at a temperature of 130°C for 14 hours. The reaction liquid was cooled to room temperature, then left overnight at room temperature, and 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 150 g; eluent, cyclohexane) to give 2.8 g of N,N'-diphenyl-N,N'-dinaphthylbenzidine (NPD) (yield 95%).

### [Example 4]

3g (30 mmol) of sodium tert-butoxide, 217 mg (0.5 mmol) of IPr-HCl (compound No. 1), 45 mg (0.2 mmol) of palladium(II) acetate, 3.94 g (20 mmol) of 2,4-dimethyldiphenylamine, 3.2 g (10 mmol) of 4,4'-dibromobiphenyl and 30 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 8 hours. The reaction liquid was cooled to room temperature, then left overnight at room temperature, and 200 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 300 g; eluent, cyclohexane/tetrahydrofuran) to give 4.95 g of N,N'-bis(2',4'-dimethylphenyl)-N,N'-diphenylbenzidine (yield 91%).

### [Example 5]

3 g (30 mmol) of sodium tert-butoxide, 434 mg (1 mmol) of IPr·HCl (compound No. 1), 90 mg (0.4 mmol) of palladium(II) acetate, 4.26 g (20 mmol) of 2-methyl-4-methoxydiphenylamine, 3.2 g (10 mmol) of 4,4'-dibromobiphenyl and 30 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 8 hours. The reaction liquid was cooled to room temperature, then left overnight at room temperature, and 200 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified by dispersing and washing with 150 ml of methanol to give 5.02 g of N,N'-bis(2'-methyl-4'-methoxyphenyl)-N,N'-diphenylbenzidine (yield 88%).

### [Example 6]

1.5 g (15 mmol) of sodium tert-butoxide, 170 mg (0.4 mmol) of IPr-HCl (compound No. 1), 45 mg (0.2 mmol) of palladium(II) acetate, 1.83 g (10 mmol) of 4-methyldiphenylamine, 1.94 g (5 mmol) of 4,4"-dibromo-p-terphenyl and 30 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 9 hours. The reaction liquid was cooled to room temperature, then left overnight at room temperature, and 200 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified by dispersing and washing with 150 ml of methanol to give 2.34 g of N,N'-diphenyl-N,N'-di-p-tolyl-4,4"-diamino-p-terphenyl (yield 79 %).

### [Example 7]

0.72 g (7.5 mmol) of sodium tert-butoxide, 180 mg (0.4 mmol) of IPr-HCl (compound No. 1), 45 mg (0.2 mmol) of palladium(II) acetate, 1.6 g (5 mmol) of bis(4-biphenyl)amine, 0.78 g (2.5 mmol) of 4,4'-dibromobiphenyl and 50 ml of toluene were put into a 100-ml three-neck flask equipped with a stirrer, a condenser tube, a thermometer and a gas-introducing duct, in an argon current at room temperature, and reacted under reflux in an oil bath controlled at a temperature of 130°C for 9 hours. The reaction liquid was cooled to room temperature, then left overnight at room temperature, and 300 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 150 g; eluent, cyclohexane/tetrahydrofuran) to give 0.97 g of N,N,N',N'-tetrakis(4-biphenylyl)benzidine (yield 49%).

### [Example 8]

1.5 g (15 mmol) of sodium tert-butoxide, 180 mg (0.5 mmol) of IMes·HCl (compound No. 9), 45 mg (0.2 mmol) of palladium(II) acetate, 1.7 g (10 mmol) of diphenylamine, 1.73 g (11 mmol) of bromobenzene and 20 ml of o-xylene were put into a 50-ml three-neck flask equipped with a stirrer, a condenser tube, a thermometer and a gas-introducing duct, in an argon current at room temperature, and reacted under reflux in an oil bath controlled at a temperature of 160°C for 7.5 hours. The reaction liquid was cooled to room temperature, and then 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 60 g; eluent, cyclohexane) to give 0.99 g of triphenylamine (yield 40%).

### [Example 9]

1.5 g (15 mmol) of sodium tert-butoxide, 240 mg (0.5 mmol) of an imidazolium derivative salt (compound No. 8), 45 mg (0.2 mmol) of palladium(II) acetate, 1.7 g (10 mmol) of diphenylamine, 1.73 g (11 mmol) of bromobenzene and 20 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 7.5 hours. The reaction liquid was cooled to room temperature, and then 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 60 g; eluent, cyclohexane) to give 1.7 g of triphenylamine (yield 69%).

### [Example 10]

1.5 g (15 mmol) of sodium tert-butoxide, 187 mg (0.5 mmol) of an imidazolium derivative salt (compound No. 11), 45 mg (0.2 mmol) of palladium(II) acetate, 1.7 g (10 mmol) of diphenylamine, 1.73 g (11 mmol) of bromobenzene and 20 ml of toluene were put into a 50-ml three-neck flask equipped with a stirrer, a condenser tube, a thermometer and a gas-introducing duct, in an argon current at room temperature, and reacted under reflux in an oil bath controlled at a temperature of 130°C for 7.5 hours. The reaction liquid was cooled to room temperature, and then 150 ml of methylene chloride was added thereto. The insoluble matter was removed by filtration, and the filtrate was washed twice with 50 ml of water. This was dewatered and dried with 30 g of anhydrous sodium sulfate, and the solvent was evaporated away to give a residue. The residue was purified through column chromatography (carrier, Fuji Silicia's NH silica gel 60 g; eluent, cyclohexane) to give 1.4 g of triphenylamine (yield 57%).

### INDUSTRIAL APPLICABILITY

According to the production method for a triarylamine compound of the invention, an industrially-applicable triarylamine compound can be produced with good production efficiency, using an inexpensive aryl halide as the starting material and using an inexpensive palladium compound as the catalyst; and the reaction may be attained under a practical condition with no additive to coexist during the reaction, and the purification operation after the reaction is simple. Since a phosphine ligand such as trialkyl phosphines is not used, it is possible to produce triarylamine compounds with reducing the burden to the environment by a phosphine ligand such as trialkyl phosphines that are phosphorus compounds.

## Claims

1. A method for producing a triarylamine compound through amination of a diarylamine compound and an aryl halide, wherein a catalyst comprising a salt of an imidazolium derivative represented by the following general formula (1): 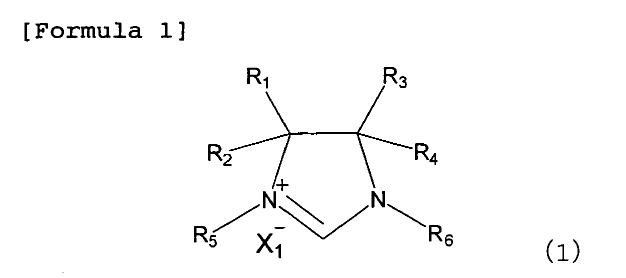 (wherein R1, R2, R3, R4, R5 and R6 may be the same or different, each representing a hydrogen atom or a hydrocarbon group having from 1 to 20 carbon atoms; X1 represents a chlorine atom, a bromine atom, an iodine atom, BF₄, CF₃SO₃ or PF₆) and a palladium compound is used, as combined with a base and a solvent to coexist therein.

2. The method for producing a triarylamine compound as claimed in claim 1, wherein the base and the solvent coexist with no other additive as combined therein.

3. A method for producing a triarylamine compound through amination of a diarylamine compound and an aryl halide, wherein a catalyst comprising a salt of an imidazolium derivative represented by the following general formula (2): 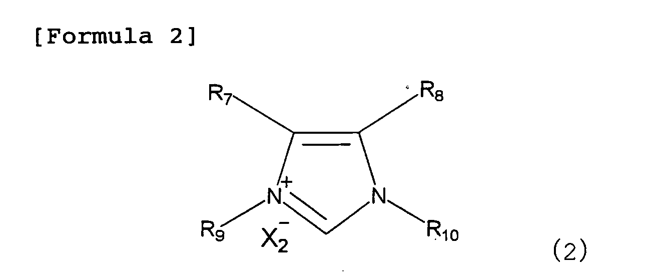 (wherein R7 and R8 may be the same or different, each representing a hydrogen atom, a hydrocarbon group having from 1 to 20 carbon atoms, or an alkoxy group having from 1 to 20 carbon atoms; R9 and R10 may be the same or different, each representing a hydrogen atom, or a hydrocarbon group having from 1 to 20 carbon atoms; R7 and R9 may form a ring, as taken together, and R8 and R10 may form a ring, as taken together; X2 represents a chlorine atom, a bromine atom, an iodine atom, BF₄, CF₃SO₃ or PF₆) and a palladium compound is used, as combined with a base and a solvent to coexist therein.

4. The method for producing a triarylamine compound as claimed in claim 3, wherein the base and the solvent coexist with no other additive as combined therein.

5. The method for producing a triarylamine compound as claimed in any of claims 1 to 4, wherein the aryl halide is an aryl bromide.

6. The method for producing a triarylamine compound as claimed in any of claims 1 to 4, wherein the diarylamine compound is a diarylamine compound having at least two amino groups.
